# EUROPEAN PATENT APPLICATION

(11) **EP 4 652 834 A1**
(43) Date of publication of application: **26.11.2025**
(21) Application number: 24177975.0
(22) Date of filing: 24.05.2024
(51) Int. Cl.: A01H 1/00, A01H 6/28, C12N 9/22, C12N 15/82

(54) **METHOD FOR PRODUCTION OF CANNABINOIDS IN PLANTS**

(71) Applicant: Quorum Biomedical S.L., 28290 Madrid (ES)
(72) Inventor: María Aizpurua de Arteche, Gonzalo, 28005 Madrid (ES); Javier Ciriza Roustan, Francisco, 28290 Madrid (ES); Sánchez Peña, Álvaro, 28005 Madrid (ES); Ruiz Cerrajero, Itziar, 28760 Madrid (ES)
(74) Representative: KIPA AB

(57) **Abstract**

Disclosed is a method of regulating production of at least one cannabinoid in plants, through the introduction of nucleotide sequences into the plant that result in one or more of: overexpression of a gene of interest to increase production of a cannabinoid of interest; enhanced production of the protein of interest by CRISPR-TAD; repression of production of competitive proteins by CRISPR-REP to promote in an indirect manner the production of the cannabinoid of interest. Also disclosed are nucleic acids for the genetic modification and plants, seeds or plant parts that result from the modification.

## Description

### FIELD

The disclosure relates to a method for improved production of cannabinoids in plants, in particular in cannabis plants. The disclosure also relates to nucleic acids, plants and vectors useful for improved production of cannabinoids in plants.

### INTRODUCTION

Cannabinoids are several structural classes of compounds that are found in plants as well as many animal organisms. More than 100 distinct cannabinoids have been isolated from cannabis. The cannabinoids are found mainly in the cannabis plant, although they have also been reported in other plants such as rhododendron, licorice, liverwort and echinacea. The most notable cannabinoids are tetrahydrocannabinol (THC), cannabidiol (CBD) and cannabinol (CBN).

The main psychoactive cannabinoid is THC, which together with CBD represents close to 40% of the extract from the cannabis plant. Both CBD and TCH have been used for their pharmaceutical activity, for example in management of appetite and weight loss from HIV/AIDS, in chemotherapy and epilepsy. Current research includes studies on the activity of various cannabinoids to establish their use for therapeutic purpose.

Lately, a high number of companies have been growing cannabis plants in order to obtain cannabinoid extracts. As the demand of different cannabinoids raises, brands and companies have covered the necessities of the market by creating new strains of *Cannabis Sativa L.,* using simple genetic techniques (such as the mendelian model), in which procedures such as cross fertilization are. This has improved the capability of companies to obtain specific amounts of each cannabinoid, by changing the plant properties.

The overexpression of the CBDAS gene and suppression of the THCAS gene through RNA interference has been reported to result in an increase by more than 10 times and a consequent 54% higher total CBD, while THC content was reduced by 37% (Deguchi, 2021). Moreover, plants grown on mine land soil (Husain et al., 2019) exhibited an 18-fold upregulation in the expression of the cannabidiolic acid synthase gene, indicating the potential for enhancing cannabinoid production in hemp through strategic cultivation practices.

There is however a need for advanced techniques for specifically regulating the production of specific cannabinoids in plants, without changing the plant genetic material in a permanent manner.

### SUMMARY

The present disclosure seeks to overcome, eliminate and/or ameliorate one or more of the drawbacks and deficiencies of the prior art, for example those described in the above.

The present disclosure generally provides nucleic acids, methods and plants for the selective production of cannabinoids.

In an aspect, the disclosure provides a method of regulating production of at least one cannabinoid in a plant, through gene expression modification of the plant, the method comprising introducing into the plant:
a first nucleotide sequence that is constructed to regulate the endogeneous transcriptional machinery of the plant to selectively increase or decrease production of a cannabinoid by the plant, the first nucleotide sequence comprising at least one guide RNA sequence directed towards a plant gene that encodes a first protein in the biosynthetic pathway of the least one cannabinoid, at least one sequence encoding an inactive Cas nuclease and at least one sequence encoding a transcriptional activator or a repressor protein, and
wherein, when the first nucleotide sequence encodes a transcriptional activator the method further comprises introducing a second nucleotide sequence into the plant that is operably linked to a promoter and encodes the first protein;
whereby the level of production of the at least one cannabinoid in the plant is regulated.

The term "directed towards a plant gene" should in the present context understood as a gene or the promoter of the gene. Thus, the at least one guide RNA sequence can be directed to the promoter of the plant gene, or the guide RNA sequence can be directed to the gene itself.

The method can further comprise introducing a third nucleotide sequence into the plant, wherein the third nucleotide sequence comprises at least one guide RNA sequence directed towards a gene that encodes a second protein in the biosynthetic pathway of the least one cannabinoid and at least one sequence encoding an inactive Cas nuclease, and wherein, when the first nucleotide sequence comprises a sequence encoding a transcriptional activator for the first protein, the third nucleotide sequence comprises a sequence encoding a transcriptional repressor for the second protein, and wherein the first nucleotide sequence comprises a sequence encoding a transcriptional repressor for the first protein, the third nucleotide sequence comprises a sequence encoding a transcriptional activator for the second protein.

Any cannabinoid can be targeted by this method. For example, the first and second proteins can be selected from CBDAS and TCHAS, so that when the first protein is CBDAS, the second protein is THCAS and when the first protein is THCAS, the second protein is CBDAS.

The disclosure also provides plants, seeds, plant explants or plant parts containing nucleic acids as described herein. Thus, in an aspect the disclosure provides a plant having an increased or decreased content of at least one cannabinoid, the plant comprising a first nucleotide sequence that is constructed to regulate the endogenous transcriptional machinery of the plant to selectively increase or decrease production of the at least one cannabinoid by the plant, the first nucleotide sequence comprising at least one guide sequence directed towards a gene that encodes a first protein in the biosynthetic pathway of the least one cannabinoid and at least one sequence encoding an inactive Cas nuclease and at least one sequence encoding a transcriptional activator or a repressor protein, and wherein, when the first nucleotide sequence is constructed to increase endogenous production of the at least one cannabinoid, the plant further comprise a second nucleotide sequence that is operably linked to a promoter and encodes the first protein in the biosynthetic pathway of the at least one cannabinoid. The plant can further comprise a third nucleotide sequence, wherein the third nucleotide sequence comprises at least one guide RNA sequence directed towards a gene that encodes a second protein in the biosynthetic pathway of the least one cannabinoid and at least one sequence encoding an inactive Cas nuclease, and
wherein, when the second nucleotide sequence comprises a sequence encoding a transcriptional activator for the first protein, the third nucleotide sequence comprises a sequence encoding a transcriptional repressor for the second protein, and wherein the second nucleotide sequence comprises a sequence encoding a transcriptional repressor for the first protein, the third nucleotide sequence comprises a sequence encoding a transcriptional activator for the second protein.The plant can be a cannabis plant, such as *Cannabis sativa*, *Cannabis indica* or *Cannabis rudelaris.*

In another aspect, the disclosure provides a nucleic acid for transforming a plant to produce increased or decreased levels of at least one cannabinoid, the nucleic acid molecule comprising (i) at least one guide RNA sequence directed to a gene encoding a first protein in the biosynthetic pathway of the at least one cannabinoid, (ii) at least one sequence encoding an inactive Cas nuclease and (iii) a first sequence encoding a transcriptional activator for the gene and/or at least one sequence encoding a transcriptional repressor for the gene.

The nucleic acid can further comprise a nucleotide sequence that comprises at least one guide RNA sequence directed towards a gene that encodes a second protein in the biosynthetic pathway of the least one cannabinoid and at least one sequence encoding an inactive Cas nuclease. Thus, when the first sequence comprises a sequence encoding a transcriptional activator for the gene encoding the first protein, the nucleic acid can further comprise a sequence encoding a transcriptional repressor for the gene encoding the second protein, and when the first sequence comprises a sequence encoding a transcriptional repressor for the gene encoding the first protein, the nucleotide sequence can further comprise a sequence encoding transcriptional activator for the gene encoding the second protein.

In this manner, the transcription of a first gene in the cannabinoid biosynthesis can be enhanced while the transcription of a second gene in the biosynthesis can be repressed, or vice versa, thereby regulating the formation of the cannabinoid.

The guide sequences in the nucleic acids and methods described herein can preferably be directed to the promoter of the gene to which they are directed.

The nucleic acid can be comprised in one or more vectors for carrying out the intended function, such as one vector, two vectors, or three or more vectors.

### BRIEF DESCRIPTION OF THE DRAWINGS

**FIG. 1** shows a graphical representation of an experimental approach for genetically transformed plants to produce altered amounts of cannabinoids.
**FIG. 2** shows a vector map ("Plasmid 2") of a vector for overexpression of CBDAS.
**FIG. 3** shows a vector map ("Plasmid 4") of a vector for activation of the transcriptional machinery.
**FIG. 4** shows a vector map ("Plasmid 5") of a vector for transcriptional repression.
**FIG. 5** shows a microscopic image of GUS detection of gene activity using a substrate that, in the presence of the GUS enzyme, produces a detectable blue reaction product under microscopy. The images show GUS staining after agroinfiltration of Cannabis sativa seedlings and leaves. Successful GUS staining was observed in the samples agroinfiltrated with the plasmid of interest.
**FIG. 6** shows in the left image a gel electrophoresis band derived from the amplification of a specific region at the *Cannabis Sativa L.* genome. The right image shows the bands resulting from the primers that recognize the specific sequence of plasmid 2, confirming this plasmid has successfully been agroinfiltrated in the sample.
**FIG. 7** shows mRNA expression of the construct genes detected by qPCR; (A) CBDAS; (B) dCAS9; (C) KRAB-COM; (D) F-Box. All figures are represented with the normalized reporter value from SYBR green reporter fluorescent signal (Rn) against PCR cycle (Ct).
**FIG. 8** shows CBDAS mRNA total levels increase after efficient agroinfiltration more than 500-fold. (A) Ct values comparing CBDAS mRNA from leaves agroinfiltrated with Control, P2 or P5 plasmids. (B) Relative expression of CBDAS mRNA with Control as the baseline. Quantification was made with 2^-ΔΔCt method.

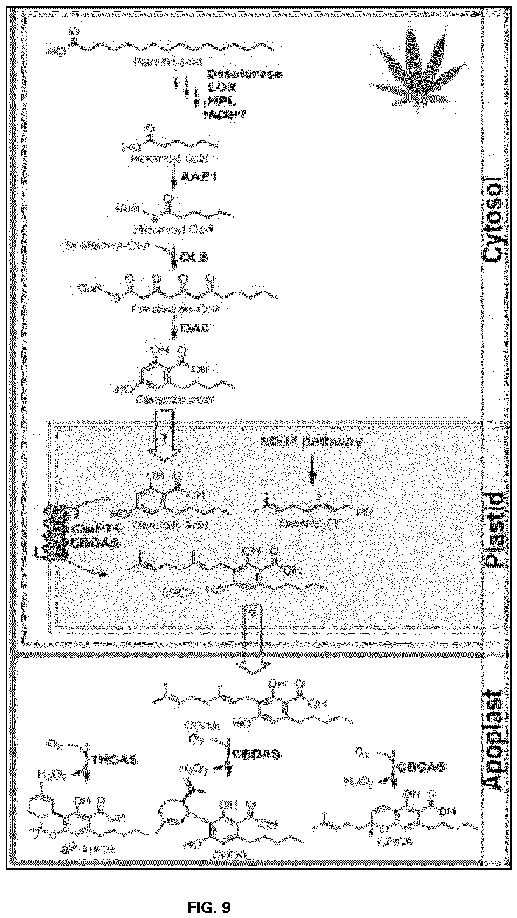

**FIG. 9** shows the subcellular distribution of enzymes in the production of cannabinoids in Cannabis Sativa. Enzymes are located in the cytosol, plastids, or apoplastic space. Question marks indicate unknown transport mechanisms. Polyketide formation takes place in the cytosol, prenylation in the plastid, and oxidocyclization and storage in the apoplast. Abbreviations: AAE1, acyl-activating enzyme 1; ADH, alcohol dehydrogenase; CBCA, cannabichromenic acid; CBCAS, cannabichromenic acid synthase; CBDA, cannabidiolic acid; CBDAS, cannabidiolic acid synthase; CBGA, cannabigerolic acid; CBGAS, cannabigerolic acid synthase; CsaPT4, C. sativa aromatic prenyltransferase 4; GA, grifolic acid; HPL, hydroperoxide lyase; LOX, lipoxygenase; MEP, methylerythritol 4-phosphate; OAC, olivetolic acid cyclase; OLS, olivetol synthase; ORS, orselinic acid synthase; RdPT1; Δ9-THCA, Δ9-tetrahydrocannabinolic acid; Δ9-THCAS, Δ9-tetrahydrocannabinolic acid synthase.

### DESCRIPTION

The present disclosure is based on the development of a method that can be used to alter cannabinoid content in plants in a controlled manner, using a combination of techniques and raw material.

The method has the following main characteristics:
- **Specificity:** the method regulates cannabinoid production selectively by modifying the cannabinoid biosynthesis pathway to promote specific increase and/or decrease of any cannabinoid.
- **Versatility:** the method application leads to different possible combinations of cannabinoids. By way of an example, the method can result in increased CBD content in a plant in a direct manner (promoting its production) and in an indirect manner by blocking THC production. By the same method, it would also be possible to only increase the production of CBD (without blocking any cannabinoid production). Production of other cannabinoids can be modified in an analogous manner.
- **Easy to use:** this application is based on different techniques that can be applied in ready-to-crop hemp cultivation.

The method is based on approaches that regulate the cannabinoids production (such as, but not limited to, THC and CBD, both together and separately) in different plants by using genetic strategies introduced in a plant such as *Cannabis sativa L.* by agroinfiltration. This can be done in a transient manner and thereby not creating a genetically modified (GMO) plant. Any agroinfiltration methods can be used in order to transfer the DNA into the plants. An advantage of the approach are the distinct genetic modifications that will be introduced into the plant.

Cannabinoids are sometimes modified upon extraction from plants by heating/combustion. For example, CBD is obtained through combustion of CBDA, and similarly THC and some other cannabinoids are obtained by heating or combustion of plant extract. In the present context therefore, discussion of cannabinoids should therefore be understood as referring to either the cannabinoid as produced by the plant or the final form of the cannabinoid as used/marketed. Thus, the cannabinoid obtained through genetic modification of a plant as described herein may be treated by heat or combustion to obtain a final cannabinoid product.

The leveraging of the CRISPR-Cas technology for simultaneous upregulation and downregulation of key genes in the cannabinoid synthesis pathway holds tremendous promise. Combining the strategies described herein, it is possible to obtain further augmentation in cannabinoid content. Thus an increase from 1.5 to 600-fold in a specific cannabinoid yield is contemplated, such as 1.5 to 300-fold, 2 to 200-fold, 5 to 200-fold, 10 to 200-fold or 20 to 200-fold, demonstrating the potential of CRISPR-based metabolic engineering for enhanced and/or downregulated cannabinoid production in plants such as *Cannabis sativa.*

The developed method includes development of methodology for genetic modification of plants as a tool for enhancing cannabinoids expression by agroinfiltration of vectors containing either enzyme genes involved in the cannabinoid biosynthetic pathway, as well as a Cas-TAD and/or Cas-Rep (e.g., dCas9-TAD and/or dCas9-Rep) that will recognize gene promoter sequences inside *Cannabis sativa L.* genome or in a cotransfected plasmid containing cannabinoid synthases enzyme sequences such as CBDAS, in order to enhance the expression of these proteins, and therefore, obtaining higher levels of cannabinoids such as cannabidiol. The method may also encompass a combination of both systems, integrating the dCas9 system as well as the sequence to be targeted, and thus amplified.

Overall, the product has different characteristics not found in other products in the market. Firstly, it is possible to produce a plant that is able to generate high levels of any cannabinoid compound without generating a genetic modified organism. Secondly, the product is flexible since it is possible to modify any part of the cannabinoid synthetic pathway and use this modification to generate different plants that overproduce any cannabinoids. Thirdly, the product is multiple since it is possible to, in one step, generate a plant that overexpresses one or more compounds or inhibits one or more enzymes in the pathway in the same plant. Thus, it is possible to change the pathway in almost infinitive ways and obtain different cannabinoids content. Finally, the product is very simple and easy-to-do so it is very likely to give benefits to the pharma companies or any company that works with cannabis plants.

The disclosed method can result in the increase or decrease of any targeted cannabinoid by 1-5% (w/w) or greater, such as by at least 2% (w/w), at least 5% (w/w), by at least 10% (w/w), by at least 15% (w/w), by at least 20% (w/w), by at least 25% (w/w), by at least 30% (w/w), by at least 35% (w/w), by at least 40% (w/w), by at least 45% (w/w) or by at least 50% (w/w).

The increase or decrease in cannabinoid production can be in the range of 5 - 50% (w/w), 10 - 50% (w/w), 10 - 45% (w/w), 10 - 40% (w/w), 15 - 40% (w/w), 20 - 40% (w/w) or 25 - 40% (w/w).

The method described herein can be applied to alter or control the production of any cannabinoid in a selective way in an analogous manner.

Exemplary cannabinoids include cannabinoids selected from cannabichromene (CBC), cannabichromenic acid (CBCA), cannabichromevarin (CBCV), cannabichromevarinic acid (CBCVA), cannabicyclol (CBL), cannabicyclolic acid (CBLA), cannabicyclovarin (CBLV), Cannabidiol monomethylether (CBDM), Cannabidiolic acid (CBDA), Cannabidiorcol (CBD-C1), cannabidivarin (CBDV), cannabidivarinic acid (CBDVA), cannabielsoic acid B (CBEA-B), cannabielsoin (CBE), cannabielsoin acid A (CBEA-A), cannabigerol (CBG), cannabigerol monomethylether (CBGM), cannabigerolic acid (CBGA), cannabigerolic acid monomethylether (CBGAM), cannabigerovarin (CBGV), cannabigerovarinic acid (CBGVA), cannabinodiol (CBND), cannabinodivarin (CBVD), cannabinol (CBN), cannabinol methylether (CBNM), cannabinol-C2 (CBN-C2), cannabinol-C4 (CBN-C4), cannabinolic acid (CBNA), cannabiorcool (CBN-C1), cannabivarin (CBV), 10-Ethoxy-9-hydroxy-delta-6a-tetrahydrocannabinol, 8,9-dihydroxy-delta-6a-tetrahydrocannabinol, cannabitriol (CBT), cannabitriolvarin (CBTV), delta-8-tetrahydrocannabinolic acid (Δ⁸-THCA), delta-9-tetrahydrocannabinol-C4 (THC-C4), Delta-9-tetrahydrocannabinolic acid A (THCA-A), delta-9-tetrahydrocannabinolic acid B (THCA-B), delta-9-tetrahydrocannabinolic acid-C4 (THCA-C4), delta-9-tetrahydrocannabiorcol (THC-C1), delta-9-tetrahydrocannabiorcolic acid (THCA-C1), delta-9-tetrahydrocannabivarin (THCV), delta-9-tetrahydrocannabivarinic acid (THCVA), 10-Oxo-delta-6a-tetrahydrocannabinol (OTHC), cannabichromanon (CBCF), cannabifuran (CBF), cannabiglendol, cannabiripsol (CBR), cannbicitran (CBT), dehydrocannabifuran (DCBF), delta-9-cis-tetrahydrocannabinol (cis-THC), trihydroxy-delta-9-tetrahydrocannabinol (triOH-THC), or a variant or analog of any of these cannabinoids.

### Genetic strategy

The genetic strategy proposed is based on three main pillars that promote several genetic modifications in order to obtain a plant expressing increased or reduced levels of a cannabinoid. The 3 main modifications will be introduced into the plant by agroinfiltration in suitable vectors, preferably in separate vectors:
- Overexpression of gene of interest to increase production of the cannabinoid of interest
- Enhance production of the protein of interest by CRISPR-TAD.
- Repress competitive proteins by CRISPR-REP to promote in an indirect manner the metabolic synthesis of the cannabinoid of choice. The repression of a protein that is a competitor of the protein of interest that produces formation of the cannabinoid of interest thereby indirectly increases production of the cannabinoid.

It should be appreciated that the method can also be used to reduce the production of any one (or several) cannabinoid, irrespective of whether this results in the increase in production of another cannabinoid. Also in this way, the overall composition of cannabinoid production by a plant can be regulated.

The different strategies above can be used in combination, i.e. incorporating all 3 modifications into the plant. Alternatively, any combination of 2 or 3 of the strategies can be used. Preferably, all three modifications are introduced to specifically increase the production of the cannabinoid of interest in a selective and efficient manner.

An example is provided by the production of elevated levels of CBD or THC. It is important to note that THC and CBD originate in the same precursor molecule called CBGA, thus, decreasing THC content or blocking its production, can lead to an increase in CBD content and vice versa. The presently disclosed method combines several techniques that increase CBD/THC content in a direct manner, promoting its production, and in an indirect manner by blocking THC/CBD production, respectively.

This can be achieved based on the ground that CBD is a molecule synthetized by CBDA synthase protein (CBDAS) (SEQUENCE ID NO.1 with mRNA predicted sequence provided in SEQUENCE ID NO.2) and THC is another cannabinoid synthetized by THCA synthase protein (THCAS) (SEQUENCE ID NO.3, with mRNA provided in SEQUENCE ID NO.4) in *Cannabis sativa L.* The proteins THCAS and CBDAS utilize the same substrate, so they compete for the substrate. In order to increase the amount of CBD content, it is possible to promote CBDAS expression and/or block THCA expression. The method provided promotes a high CBD content plant by two independent pathways, the activation of CBDAS expression (endogenous and/or exogenous) and repression of THCAS expression. This way, a plant that produces increased levels of CBD and decreased levels of THC can be produced.

The method can also be applied in an inverse manner to create a high THC content plant with, i.e. by increasing expression of THCAS and repressing expression of CBDAS.

The general method is based on the dCas9-TAD (transcriptional activator domain) system. Although exemplified herein to increase CBDA content the method can be translated to other genes involved in the cannabinoid biosynthesis pathway. The system is based on the possibility of targeting a gene or multiple genes in a specific way using the CRISPR/Cas system and the use of transcription activators that can modulate the expression of a gene and therefore, the protein content. The goal is to obtain a non-transgenic plant that overexpresses the enzyme that synthesizes a cannabinoid of interest, such as CBDA. The system achieves its goal not only due to the specific targeting of a specific promoter to increase the production of the cannabinoid (such as the CBDAS promoter), but also, or alternatively, the expression activation promoted by TADs. The system has 3 main components
1. **Inactive Cas nuclease:** CRISPR/Cas is a system based on the "immunity" or defense strategies that bacteria have in order to avoid bacteriophages attack. One of the main elements of this system is the Cas nuclease, which is a double strand nuclease, that using a single-guide RNA (sgRNA) can make a double strand break in the genome in a specific region of the genome. Several types of Cas proteins have been discovered but the one that is widely used is Cas9. This nuclease is DNA specific and introduces a double strand break (DSB) inside the genome, making it a perfect tool for genome editing. However, there are other variations of Cas9 such as dCas9 (dead Cas9), which has 2 mutations: H840A in the HNH domain and D10A in the RuvC domain. These mutations block the nuclease activity; therefore, this protein will only bind to a specific target, but it will not have another activity. The advantage of dCas9 is that it recognizes specific loci in the genome, but it will not edit (cleave) the loci. Thus, by creating a fusion protein formed by dCas9 and another protein, it is possible to locate any desired protein in a specific locus of a plant genome, such as *Cannabis sativa* genome. It should be noted that any other inactive form of a Cas nuclease can be used, as long as the Cas nuclease variant is lacking its nuclease activity. Accordingly, in the present context, the term "inactive Cas nuclease" refers to a Cas nuclease that lacks nuclease activity. A number of stable dCas9 have been generated for this reason and represent potential alternative Cas proteins that can be used, including pcoCas9 (plant codon-optimized Cas9), dCas9p (plant codon-optimized Cas9 harboring enriched guanine-cytosine content with Key 5'coding regions), etc. These proteins all have several point modifications that make the proteins more stable and/or effective.
   Alternatively, other suitable nucleases can be used in the methods described herein, such as CPF1 nuclease.
2. **sgRNA:** sgRNAs (single-guided RNA, also called guide RNA) are also an element of the CRISPR/Cas system which is indispensable for the specificity property that defines it. This RNA is formed by a nucleic specific sequence that will be complementary to the DNA target at the genome and a tracrRNA that is necessary for the Cas nuclease (e.g., Cas9 or dCas9) attachment. In a normal system, this RNA will interact thanks to complementary bases to the target in the genome and Cas9 will recognize it and cut the genome in a specific locus. If dCas9 is used, a genome locus will be recognized but not cut. Another advantage of this system is that it allows use of multiple sgRNAs in order to target two or more loci in the genome. Thus, in certain embodiments, two or more sgRNAs are used to target two or more loci. This way it is possible to target multiple genes. By way of example, this way not only CBDAS could be targeted but also THCAS. Thus, if the goal is having an increased ratio of certain cannabinoids such as increased CBD/THC ratio, it is possible to target the two genes, thereby blocking THCAS transcription (by dCas9 and repressor protein fusion) and enhancing CBDAS expression (by dCas9-TAD system, see below) at the same moment in the same plant.

Alternatively, the system could be implanted in an inverse fashion to decrease the CBD/THC ratio, by blocking CBDAS transcription and enhancing THCAS expression.

It may be advantageous to use multiple sgRNA targeting the same promoter. Thus, even though one guide RNA is enough for directing dCas9 to the target, it may be advantageous to use multiple sgRNAs (two or more, such as 2, 3 or 4 sgRNAs) for improving efficiency. For example, sgRNA can target CBDAS promoter localized in Chr7 of *Cannabis sativa* (at 9:30982769-30983071 at ensemblplants.es). The sgRNA should have suitable sequence, such as a PAM sequence, which is a 3-nucleotide sequence (NGG) that must recognize Cas9 to bind the target DNA. sgRNA sequences can be specific-site mutated to gain more RNA stability, higher dCas9 recognition capacity and reducing off-targets (similar genome sequences that can be also recognized by this system). Examples of sgRNA sequences is shown in SEQ ID NO:5 - 19, although the skilled person will appreciate that other or alternate sgRNAs are can be selected depending on the intended role.

3. **TAD:** transcriptional activator domains are parts of different proteins found in several organisms such as bacteria or yeast that show great abilities in enhancing gene transcription. These transcriptional activators are usually found in physiological proteins that regulate the transcription of the genes. These proteins act in the promoter region, where gene transcription is regulated. The disclosed system can therefore include such TADs that, in combination with features of the CRISPR/dCas9 system will enhance gene expression (for example, of CBDAS or any other gene involved in cannabinoid synthesis). For this purpose a Cas protein (such as dCas9) can be fused to a TAD guided by sgRNAs to the promoter of the gene of interest, i.e. a gene that is involved in cannabinoid synthesis (such as CBDAS). A wide variety of TADs are known and have been studied in plants. Any such TAD can be used in the method disclosed herein. Exemplary TADs include AD, AAD, PRD, QAD, STAD, leucine zipper domain, bZIP, zinc finger domain, helix-loop-helix domain, steroid hormone receptor domain, TATA-box binding protein associated factor (TAF) domain, nuclear receptor activation function (AF) domain, VP64, p65, Rta, HSF1, VPR (e.g., VP64, p65, Rta), PH, EDLL, VP16, VP128, TAL and HAT

Any of these TADs can be used when fused to dCas9 or to gRNA binding proteins in order to enhance the expression of a specific protein of interest.

Also, these fusion proteins can contain more than one TAD, such as two TADs or three TADs. For example, one of the most powerful tools used in plants with this system is what is called dCas9-TV, formed by dCas9 fused to VP128 and 6 TAL, that can produce an overexpression of more than a thousand fold in *Oryza sativa.* Any combination of TADs can be used, including any combination of the TADs mentioned in the foregoing (e.g., EDLL, VP16, VP128, VP64, TAL)

It is also possible to join the TAD to a guide RNA (sgRNA). For this purpose, an additional RNA loop can be introduced in the sgRNA that can be recognized by a recognition protein. One such recognition protein is MS2 coat protein, although other suitable recognition proteins are also contemplated. The recognition protein also can be fused to other TAD or multiple TADs in order to enhance gene expression in a sgRNA-specific manner. Moreover, this system allows targeting of multiple cannabinoid genes at the same time. Such a system has been shown to promote a significant increase in gene expression, e.g. a 1000-fold or a 1500-fold or more increased gene expression in *Arabidopsis.*

The method can be adapted to include a repressor rather than a transcriptional activator. This way, the production of specific proteins can be repressed, for example proteins that compete with the production of the cannabinoid of interest. Thus, either by repressing protein production, or by repressing protein production in combination with specific transcriptional activation of a second protein involved in the production of the cannabinoid, an effective system for producing the cannabinoid can be generated.

Such a fusion protein consisting of dCas9 and repressor protein (dCas9-Rep) can thus be used instead of, or in combination with, a dCas9-TAD. Any suitable repressor that can work within such fusion protein can be used. One example is SRDX; this domain is derived from the transcriptional repressor domain referred to as the ERF-associated amphiphilic repression domain (EAR). It is a potent plant repression domain even when it is co-expressed with other activators such as VP16. Another suitable repressor is KRAB. Other possible repressors include DLN144, DLS, KYP, BRD and MIX (Xu et al, Gene, 2023, 851, 146967).

This system can thus be synergically used with a system enhancing gene expression. For example, THCAS and CBDAS utilize the same substrate, so these proteins compete for the substrate. In order to increase the amount of CBD content, CBDAS expression can be promoted and/or block THCAS expression. This way it is possible to promote a high CBD content in a plant by two independent pathways.

Alternatively, the expression of CBDAS can be repressed and expression of THCAS promoted. This way, a high THC content in the plant is promoted. The same methodology can be used for any cannabinoids that result from competition for the same substrate by two different proteins in the cannabinoid biosynthetic pathway.

It is possible to use the above described solutions in combination. For example, the different constructs can be introduced in different vectors that can then be introduced into a suitable plant to promote the specific formation of one or more cannabinoid. In such a system, the production of one or more cannabinoid can be specifically enhanced and/or the production of one or more cannabinoid repressed. The main idea is to produce non-transgenic or non-genetically modified plants, such as *Cannabis sativa* plants that are engineered to produce specific cannabinoids in a controlled manner. For this purpose, the combination of any one or the combination of the above 3 elements can be utilized. The best combination of TADs and/or repressors can be used to achieve this goal.

A system introducing all three features can be defined as dCas9-TAD-IP-CBDAS or dCas9-TIC. Such a system contains components within a system that can overexpress any gene from the cannabinoid biosynthesis pathway thanks to a cotransfection of dCas9-TAD system and a plasmid containing the gene with a known promoter. Importantly, the system can also be used for the overexpression of other cannabinoid compounds, since the method can be altered in the same way in order to target one gene or another (or combination of both). Thus, by way of example, instead of targeting the CBDAS promoter to produce a plant with high levels of CBD, the THCAS promoter can be targeted to produce a plant with high THC levels.

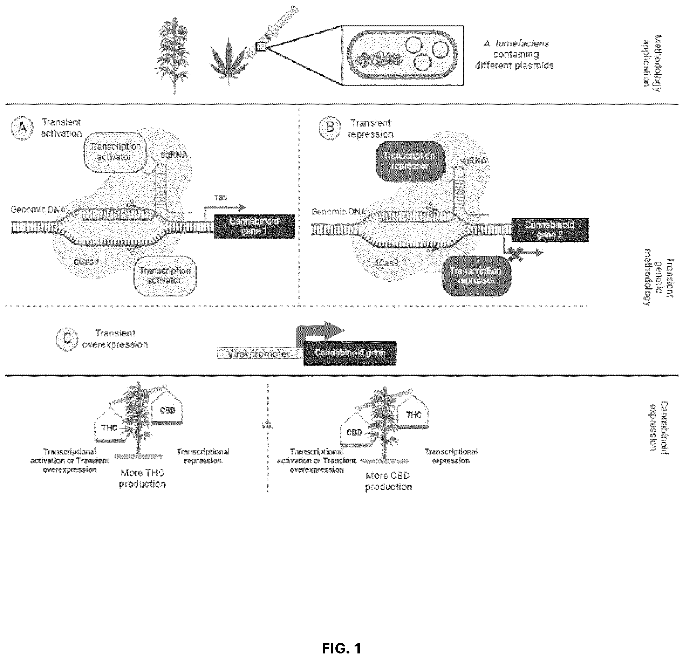

FIG. 1 provides a graphical representation of the experimental approach in accordance with the current disclosure, showing how introductions of three different vectors into Cannabis sativa plants can be used to alter its cannabinoid content. A first vector (A) aims to promote transcriptional activation of a cannabinoid gene by the dCas9 system. A second vector (B) aims to promote transcriptional repression of a cannabinoid gene by the dCas9 system. A third vector (C) aims to promote a cannabinoid gene overexpression. The result of this technology is a plant with different cannabinoid production such as more CBD content, more THC content, or more or altered contents of other cannabinoids.

It will be appreciated that the above approach can be used to engineer any suitable plant species to produce one or more cannabinoid species. The plant can in some embodiments be a Cannabis plant, such as *Cannabis sativa*, *Cannabis indica* or *Cannabis ruderalis.*

### Agroinfiltration

The nucleic acids described herein can be introduced into plants using for example agroinfiltration.

Agroinfiltration is a delivery method for introducing nucleic acid constructs into plants. The method, also called agrobacterium infiltration, is based on the bacteria *Agrobacterium tumefaciens*, as these bacteria have a singular property: they are able to successfully deliver a plasmid (molecule of extrachromosomal DNA) in plants. The plasmid is known under the name of "**Ti plasmid**" which stands for Tumor inducing plasmid, due to the original properties of the plasmid for inducing tumorigenesis in plants. This property allows the delivery of small portions of DNA into plant cells, without creating a stable transgenic, since the DNA can stay extrachromosomal. This means a transgenic organism would not result from this genetic delivery, as the plasmid stays in the nucleus but does not interfere with the plant genome and does not transfer to heritage.

Any suitable *Agrobacterium* strain can be used. Exemplary strains include GV3101, EHA101, LBA4404, AGL1. It has previously been shown that the GV3101 strain is optimal for *C. sativa* agroinfiltration (Deguchi *et al.*, 2020).

The *A. tumefaciens* Ti plasmid can be genetically altered, exchanging its tumor-inducing genes with a specific sequence of interest (T-DNA). Thus, introducing a sequence in the plasmid will result in specific protein/enzyme production in a short period of time. In this case, the plasmid needs to be designed and assembled. By applying this method for the introduction of cannabinoids synthases in *C. sativa L.*, it is possible to promote not only high levels of cannabinoids compounds but also reduce the time of plant harvesting. This in turn results in the benefit of obtaining valuable cannabinoids such as THC and CBD, among others, in a rapid and efficient manner.

The levels of protein production with agroinfiltration mediated protein expression surpasses the amounts obtained with stable transgenic plants, mainly due to the suppression of the position effect produced by integrated transgenic material. Thus, agroinfiltration results in superior protein expression.

For protein production mediated through agroinfiltration, a specific construct must first be designed and created, such as constructs as described herein. This construct therefore contains a sequence of interest (in one example, the sequence for CBD Acidic Synthase (CBDAS)). For the design of the construct, any suitable vector backbone can be used. Exemplary backbones are pGreen, pGreen-like and pCAMBIA vectors. The desired constructs, explained in the above, can subsequently be introduced into the backbone vector.

Vectors that include dCas9 technology explained herein can use any suitable backbone known in the art. For example, the backbone may be selected from pBI, pBIN, pCAMBIA, pEarlygate, pFGC, pGA, pGREEN, pGREEN-like, PGREEN II, pHANNIBAL, pHELLSGATE, pKANNIBAL, pPZP, pRI, pSB, pYPQ, pHSN and derivatives thereof. One suitable backbone is pGreen-like. This backbone allows transformation of larger plasmids inside bacteria that already contain a helper vector called pSoup. pGreen-like vectors contain only a part of the origin of replication that is only useful when the other part is present in pSoup vectors. Specifically, pSoup vectors contain a protein called RepA which is imprescindible for the recognition of pSa as the origin for replication inside Agrobacterium Tumefaciens.

Other suitable vectors could be used. The vector can for example be based on pCAMBIA which contains all the elements for successful agroinfiltration but its length is quite big. In agroinfiltration, all the genetic material of interest you want to be expressed in plants must be flanked by Left and Right border sequences (LB and RB). These sequences come from the original Ti plasmid and they play a key role in the genetic delivery process, as the sequence contained between them will be the one to be inserted in the nucleus, thus replicated once the cells are successfully infected. There are other suitable strategies for gene expression, such as pEAQ and pBINPLUS, as well as the Cowpea Mosaic Virus RNA2.

An optimization of the expression vector must be performed. As every gene needs a promoter for it to be transcribed into mRNA, a suitable promoter for the particular genetic sequence must be chosen. Suitable promoters include constitutive promoters, synthetic promoters, inducible promoters, tissue specific promoters, etc. Examples of suitable promoters include Act1, Adh1, Hsp18.2, ScBV, UVI-1, RUBQ1/RUBQ2, Gmubi, Nos, CmYLCV, KST1, Cula11/Cula08, P OsCon1, Tctp, etc. Another example is the *Cauliflower Mosaic Virus promoter* (CaMV 35S), which is suitable due to the already proven efficiency of this virus promoter in plants.

A suitable transcriptional terminator is also needed. Such terminators are known in the art, for example Tobacco Mosaic Virus Terminator (TMV Terminator), CaMV 35S Terminator, Octopine Synthase Terminator (OCS Terminator), Pinll Terminator, t35S Terminator, tHSP18, t35S, tOcs and Nopaline terminator (tNos).

It may be advantageous to add a gene silencing repressor, such as p19, to help to achieve higher yields of transcriptional activity. Repressors include RNA silencing suppressors, such as P19 (Tomato bushy stunt virus protein 19), HC-Pro (Helper-component proteinase) from Potato virus Y, viral proteins such as 2b from Cucumber mosaic virus (CMV), P1 from Tobacco mosaic virus, P25 from Tobacco mosaic virus and non-viral proteins such as P1/HC-Pro (hybrid of P1 and HC-Pro) and HcPro-P19 (hybrid of HC-Pro and P19).

Such gene silencing repressors do not necessarily need to be part of the expression vector. Thus, although the gene silencing repressor can be suitably part of the gene expression vector, the repressor can alternatively be introduced by co-infiltration method.

The expression vector (i.e., the substitute for the original Ti plasmid) must be introduced in *Agrobacterium tumefaciens.* As with any other bacterial transformation with plasmids, the two most wide-spread techniques are electroporation and heat-shock methods. In order to be able to select the correctly transformed bacteria, antibiotic resistance genes can be introduced inside the plasmid, which means that the bacteria that have been correctly transformed will be able to grow in media that contains this specific antibiotic, whereas bacteria that have not been successfully transformed will not be able to survive the antibiotic conditions. The last step of this process will then be to inoculate the transformed bacterial culture product of the steps described above, into the targeted plants.

In one specific method, *Agrobacterium Tumefaciens* can be used together with a vector based on pSoup and p19 as a gene silencing repressor that are chemocompetent for heat shock transformation.

In agrobacterium infiltration, a suspension culture of *Agrobacterium tumefaciens* cells is infiltrated into the organs of an intact plant. This provides a rapid and efficient way to express foreign genes in the plant. Several agroinfiltration methods have been developed, including:
Vacuum system - this entails the submersion of the plant tissue into a liquid suspension of *A. tumefaciens* which is subjected to decreased pressure followed by rapid repressurization. This one is a common method for introducing bacteria to the interior of the plant tissue. It has been successfully employed for the genetic transformation of several plants.
Syringe infiltration is the most popular method for agroinfiltration and is widely used in many molecular studies. It consists of a simple procedure where a needleless syringe is used to introduce *Agrobacterium* into plant leaves, with no need for specialized equipment and making it the simplest and most efficient method of agroinfiltration for gene function analysis. The method has many advantages and can either transfer one target gene into the whole leaf, or introduce multiple genes into different areas of one leaf, allowing multiple assays to be conducted on a single leaf. This facilitates the optimization of experimental parameters that potentially influence the protein expression efficiency. Although only a few plant species are naturally amenable to syringe infiltration, it has been successfully applied to *Arabidopsis*, tobacco, onion, potato, citrus, tomato, grape, and lettuce.
Agrobacterium submersion consists of introducing the whole plant or explants in the Agrobacterium suspension. The correct media needs to be used, harboring the bacteria and different surfactant compounds that facilitate the introduction of the Agrobacterium inside the plant host cells.
Agrobacterium spray entails spraying either the whole plant or regions of interest with transformed agrobacterium for genetic delivery. However, it needs previous plant treatment with surfactants that will allow the plant to take up bacteria. This method can be especially useful since it is the one that offers more benefits to the user when application of the treatment is performed, due to the ease of the procedure and the simplicity of the technology needed for it.

### Genetically engineered plants

The methodology described herein can be used to produce plants that have been engineered to produce increased or decreased levels of certain cannabinoids. Such non-GMO plants, such as genetically engineered *Cannabis sativa* plants have the unique advantages as described herein of allowing selective production of any cannabinoid.

The disclosure by extension also provides such plants. The disclosure also provides seeds, cells, explant or parts of such genetically modified plants.

### Advantages of the invention

The current state of the art with respect to genetic engineering for the production of cannabinoids in *Cannabis sativa* is focused on inserting plant-foreign genes. By contrast the approach disclosed herein differs in that the enzymes to be inserted are endogenous of *Cannabis sativa L.* Thus, signals for deglycosylation, endoplasmic reticulum signals (such as KDEL or HDEL), purification tag sequence or any other sequence needed for the specific procedure that the use of a different plant species may require are generally not to be part of the construct. This is thanks to the endogenous character of the enzymes to be produced, post translational modifications are carried out by the plant itself. In addition to this, other features differ from one to another, as the technique proposed by the actual state of the art can result in host plant toxicity, as foreign genes are to be inserted in the plants of choice. Again, there is a distinct advantage of the presently disclosed method in that there is no risk of plant toxicity as the genes to be inserted are endogenous.

In addition, it has been shown that CBD works better with a little amount of THC present, instead of CBD purified alone. Hence, generating a cannabinoid such as CBD in *C. sativa* will have increased quality since an endogenous level of THC will also be produced. This property is not found if CBD is specifically produced in other systems rather than *C. sativa.* Furthermore, the construct described herein may also include a downregulation mechanism for the competitor enzyme. Thus, as an example, if genes for the magnification of the CBD biosynthetic pathway are introduced, downregulators for THC production enzymes (which may imply THCAS) will be added in the procedure, as these enzymes are competitive for the same substrate.

The described procedure can be extended to other enzymes involved in cannabinoid biosynthetic pathway, such as CsTKS, CsOAC, CsPT4, AAE1 or AAE3 among others, which have also been proved to be crucial in the cannabinoid pathway (Fig. 9). In addition to this, different combinations of all the enzymes involved in the cannabinoid pathway will be tested to determine the best combination for cannabinoid production, added to the knockdown of other cannabinoid enzymes using specific gRNAs to promote even more plant cannabinoid production specificity.

It should be noted that the presently described method can be applied to either generate transiently modified plants or to generate stable genetically modified organisms (GMOs). Therefore, although the examples provided herein relate mainly to a method involving transient modifications to the cannabis plant, which does not result in the creation of genetically modified organisms (GMOs), it is important to note that the techniques and methodologies detailed herein are adaptable and could be modified to facilitate stable genetic alterations. By employing such stable modifications, the method could then be utilized to produce genetically modified cannabis plants, thus broadening the applicability and utility of the disclosed process. This adaptability allows for potential expansion in the scope of the method to include GMO variants, offering opportunities for further development and application in accordance with regulatory approvals.

For example, if the main aim is to create a stable genetically modified plant that expresses the genetic changes described herein, the methodology to follow could consist of introduction of designed nucleotide sequences into agrobacterium as described, including also a genetic sequence that codifies for an antibiotic resistance gene. After agroinfiltration of the plant (or plant cells/explants/protoplast/seeds/other plant tissue), antibiotic selection can be performed to detect those that have incorporated the genetic modifications into the plant genome. Subsequently, the plant (or plant tissue) can be regenerated in order to obtain a genetically modified plant. This way the methodology described herein can be used to create modified cannabis plants with the desired cannabinoid yield.

As should be apparent from the description provided herein, some advantages of the presently disclosed method and system include:
- **Novelty:** the genetic method is suitable to alter cannabinoid quantity in a specific and customizable manner. It combines the CRISPR genetic method combination plus interference to generate a transient or stable genetic modification.
- **Innovation:** through the disclosed method it is possible to obtain a specific quantity of cannabinoid in an elevated manner. The easy application of the method and its versatility makes it totally innovative in the field of cannabinoid production
- **Unique combination strategy approach:** the disclosed method combines different strategies in a single and uniquely viable method. The method consists of selective genetic modifications specifically directed to the synthetic route of the cannabinoid compounds, in addition to altering specifically cannabinoid quantity.
- Versatility: among the numerous applications that the method possesses, one of them is characterized by the selective and multiple modifications that can be done in the cannabinoid pathway. Specifically, using more than one gRNA directed to different genes within the pathway, the disclosed method is able to alter different points (i.e. expression of different proteins) simultaneously in the pathway leading to a better regulation of the cannabinoids products in the plant.

Non-limiting examples of the embodiments of the present disclosure include:
1.A method of regulating production of at least one cannabinoid in a plant, through gene expression modification of the plant, the method comprising introducing into the plant:
   a first nucleotide sequence that is constructed to regulate the endogenous transcriptional machinery of the plant to selectively increase or decrease production of a cannabinoid by the plant, the first nucleotide sequence comprising at least one guide RNA sequence directed towards a plant gene that encodes a first protein in the biosynthetic pathway of the least one cannabinoid, at least one sequence encoding an inactive Cas nuclease and at least one sequence encoding a transcriptional activator or a repressor protein, and
   wherein, when the first nucleotide sequence encodes a transcriptional activator the method further comprises introducing a second nucleotide sequence into the plant that is operably linked to a promoter and encodes the first protein;
   whereby the level of production of the at least one cannabinoid in the plant is regulated.
2. The method of paragraph 1, the method further comprising introducing a third nucleotide sequence into the plant, wherein the third nucleotide sequence comprises at least one guide RNA sequence directed towards a gene that encodes a second protein in the biosynthetic pathway of the least one cannabinoid and at least one sequence encoding an inactive Cas nuclease,
   wherein, when the first nucleotide sequence comprises a sequence encoding a transcriptional activator for the first protein, the third nucleotide sequence comprises a sequence encoding a transcriptional repressor for the second protein, and wherein
   when the first nucleotide sequence comprises a sequence encoding a transcriptional repressor for the first protein, the third nucleotide sequence comprises a sequence encoding a transcriptional activator for the second protein.
3. The method of any of the previous paragraphs, wherein the plant is a cannabis plant.
4. The method of the previous paragraph, wherein the cannabis plant is selected from *Cannabis sativa, Cannabis indica* and *Cannabis rudelaris.*
5. The method of any one of the previous paragraphs, wherein the inactive Cas nuclease is dCas9.
6. The method of any of the previous paragraphs, wherein the sequence encoding a transcriptional activator and/or repressor is fused to the sequence encoding an inactive Cas nuclease and/or a guide RNA binding protein sequence.
7. The method of any one of the previous paragraphs, wherein the transcriptional activator is selected from AD, AAD, PRD, QAD, STAD, leucine zipper domain, bZIP, zinc finger domain, helix-loop-helix domain, steroid hormone receptor domain, TATA-box binding protein associated factor (TAF) domain, nuclear receptor activation function (AF) domain, VP64, p65, Rta, HSF1, VPR (e.g., VP64, p65, Rta), PH, EDLL, VP16, VP128, TAL,HAT and any combinations thereof.
8. The method of any one of the previous paragraphs 2-7, wherein the transcriptional repressor is selected from SRDX-domain containing repressor, a KRAB-domain containing repressor, DLN144, DLS, KYP, BRD and MIX.
9. The method of any one of the previous paragraphs, wherein the first and/or third nucleotide sequence further comprises at least one guide RNA sequence.
10. The method of any one of the previous paragraphs, wherein the first and/or third nucleotide sequence further comprises at least two distinct guide RNA sequences.
11. The method of any one of the previous two paragraphs, wherein the at least one guide RNA sequence encodes an RNA sequence that can be recognized by a recognition protein, such as MS2 coat protein or a COM protein.
12. The method of any one of the previous three paragraphs, wherein the transcriptional activator is fused to a protein that specifically recognizes the at least one guide RNA, such as MS2 coat protein.
13. The method of any one of the previous four paragraphs, wherein the transcriptional repressor is fused to a protein that specifically recognizes the at least one guide RNA, such as a COM protein.
14. The method of any one of the previous paragraphs, wherein the first protein is CBDAS or THCAS.
15. The method of any one of the previous paragraphs, wherein when the first protein is CBDAS, the second protein is THCAS and when the first protein is THCAS, the second protein is CBDAS.
16. The method of any one of the previous paragraphs, wherein the repressor or the activator targets the transcription of endogenous THCAS or CBDAS.
17. The method of any one of the previous paragraphs, wherein the level of production of the at least one cannabinoid is increased or decreased by at least 5% (w/w), by at least 10% (w/w), by at least 15% (w/w), by at least 20% (w/w), by at least 25% (w/w) or by at least 30% (w/w), compared with the endogenous production of the at least one cannabinoid.
18. The method of the previous paragraph, wherein the level of production of the least one cannabinoid is increased or decreased by up to 30%, up to 35% or up to 40% (w/w).
19. The method of any one of the previous paragraphs, wherein the cannabinoid is selected from cannabichromene (CBC), cannabichromenic acid (CBCA), cannabichromevarin (CBCV), cannabichromevarinic acid (CBCVA), cannabicyclol (CBL), cannabicyclolic acid (CBLA), cannabicyclovarin (CBLV), Cannabidiol monomethylether (CBDM), Cannabidiolic acid (CBDA), Cannabidiorcol (CBD-C1), cannabidivarin (CBDV), cannabidivarinic acid (CBDVA), cannabielsoic acid B (CBEA-B), cannabielsoin (CBE), cannabielsoin acid A (CBEA-A), cannabigerol (CBG), cannabigerol monomethylether (CBGM), cannabigerolic acid (CBGA), cannabigerolic acid monomethylether (CBGAM), cannabigerovarin (CBGV), cannabigerovarinic acid (CBGVA), cannabinodiol (CBND), cannabinodivarin (CBVD), cannabinol (CBN), cannabinol methylether (CBNM), cannabinol-C2 (CBN-C2), cannabinol-C4 (CBN-C4), cannabinolic acid (CBNA), cannabiorcool (CBN-C1), cannabivarin (CBV), 10-Ethoxy-9-hydroxy-delta-6a-tetrahydrocannabinol, 8,9-dihydroxy-delta-6a-tetrahydrocannabinol, cannabitriol (CBT), cannabitriolvarin (CBTV), delta-8-tetrahydrocannabinolic acid (Δ⁸-THCA), delta-9-tetrahydrocannabinol-C4 (THC-C4), Delta-9-tetrahydrocannabinolic acid A (THCA-A), delta-9-tetrahydrocannabinolic acid B (THCA-B), delta-9-tetrahydrocannabinolic acid-C4 (THCA-C4), delta-9-tetrahydrocannabiorcol (THC-C1), delta-9-tetrahydrocannabiorcolic acid (THCA-C1), delta-9-tetrahydrocannabivarin (THCV), delta-9-tetrahydrocannabivarinic acid (THCVA), 10-Oxo-delta-6a-tetrahydrocannabinol (OTHC), cannabichromanon (CBCF), cannabifuran (CBF), cannabiglendol, cannabiripsol (CBR), cannbicitran (CBT), dehydrocannabifuran (DCBF), delta-9-cis-tetrahydrocannabinol (cis-THC), trihydroxy-delta-9-tetrahydrocannabinol (triOH-THC), or a variant or analog of any of these cannabinoids.
20. A plant having an increased or decreased content of at least one cannabinoid, the plant comprising
   a first nucleotide sequence that is constructed to regulate the endogenous transcriptional machinery of the plant to selectively increase or decrease production of the at least one cannabinoid by the plant, the first nucleotide sequence comprising:
   at least one guide sequence directed towards a promoter region of a gene that encodes a first protein in the biosynthetic pathway of the least one cannabinoid and at least one sequence encoding an inactive Cas nuclease and at least one sequence encoding a transcriptional activator or a repressor protein, and
   wherein, when the first nucleotide sequence is constructed to increase endogenous production of the at least one cannabinoid, the plant further comprise a second nucleotide sequence that is operably linked to a promoter and encodes a first protein in the biosynthetic pathway of the at least one cannabinoid.
21. The plant of the previous paragraph, wherein the plant further comprises a third nucleotide sequence, wherein the third nucleotide sequence comprises at least one guide RNA sequence directed towards a gene that encodes a second protein in the biosynthetic pathway of the least one cannabinoid and at least one sequence encoding an inactive Cas nuclease, and
   wherein, when the second nucleotide sequence comprises a sequence encoding a transcriptional activator for the first protein, the third nucleotide sequence comprises a sequence encoding a transcriptional repressor for the second protein, and wherein the second nucleotide sequence comprises a sequence encoding a transcriptional repressor for the first protein, the third nucleotide sequence comprises a sequence encoding a transcriptional activator for the second protein.
22. The plant of any one of the previous two paragraphs, wherein the plant is a cannabis plant.
23. The plant of any one of the previous three paragraphs, wherein the plant is *cannabis sativa.*
24. A nucleic acid for transforming a plant to produce increased or decreased levels of at least one cannabinoid, the nucleic acid comprising (i) at least one guide RNA sequence directed to a gene encoding a first protein in the biosynthetic pathway of the at least one cannabinoid, (ii) at least one sequence encoding an inactive Cas nuclease and (iii) a first nucleotide sequence encoding a transcriptional activator for the gene and/or at least one sequence encoding a transcriptional repressor for the gene, and
   wherein, when the first nucleotide sequence encodes a transcriptional activator the nucleic acid further comprises a second nucleotide sequence that is operably linked to a promoter and encodes the first protein.
25. The nucleic acid of the previous paragraph, further comprising a nucleotide sequence that comprises at least one guide RNA sequence directed towards a gene that encodes a second protein in the biosynthetic pathway of the least one cannabinoid and at least one sequence encoding an inactive Cas nuclease, and
   wherein, when the first sequence comprises a sequence encoding a transcriptional activator for the gene encoding the first protein, the nucleic acid further comprises a sequence encoding transcriptional repressor for the gene encoding the second protein,
   and when the first sequence comprises a sequence encoding a transcriptional repressor for the gene encoding the first protein, the nucleic acid further comprises a sequence encoding transcriptional activator for the gene encoding the second protein.
26. The nucleic acid of any one of the previous two paragraphs, wherein the at least one sequence encoding an inactive Cas nuclease is fused to the at least one sequence encoding a transcriptional activator or the transcriptional repressor so as to encode a Cas nuclease fusion protein.
27. The nucleic acid of any one of the previous three paragraphs, wherein the transcriptional activator is selected from AD, AAD, PRD, QAD, STAD, leucine zipper domain, bZIP, zinc finger domain, helix-loop-helix domain, steroid hormone receptor domain, TATA-box binding protein associated factor (TAF) domain, nuclear receptor activation function (AF) domain, VP64, p65, Rta, HSF1, VPR (e.g., VP64, p65, Rta), PH, EDLL, VP16, VP128, TAL and HAT, and any combinations thereof.
28. The nucleic acid of any one of the previous four paragraphs wherein the transcriptional repressor is selected from SRDX-domain containing repressor, a KRAB-domain containing repressor, DLN144, DLS, KYP, BRD and MIX
29. The nucleic acid of any one of the previous four paragraphs, wherein the at least one guide RNA sequence contains an RNA sequence that is recognized by a protein that is fused to a transcriptional activator or transcriptional repressor, to thereby enhance or reduce recruitment of the transcriptional machinery.
30. The nucleic acid of any one of the previous five paragraphs, wherein the transcriptional activator is fused to a protein that recognizes the at least one guide RNA and enhances recruitment of transcriptional activators, such as MS2 coat protein.
31. The nucleic acid of any one of the previous six paragraphs, wherein the transcriptional repressor is fused to a protein that specifically recognizes the at least one guide RNA, such as a COM protein.
32. The nucleic acid of any one of the previous paragraphs 26 - 31, wherein the nucleic acid is provided in one or more vectors.
33. The nucleic acid of the previous paragraph, wherein a first vector comprises (i) at least one guide RNA sequence directed to a gene encoding a first protein in the biosynthetic pathway of the at least one cannabinoid, (ii) at least one sequence encoding an inactive Cas nuclease and (iii) a first sequence encoding a transcriptional activator for the gene and/or at least one sequence encoding a transcriptional repressor for the gene.
34. The nucleic acid of paragraph 33, wherein a second vector comprises a nucleotide sequence that is operably linked to a promoter and encodes the first protein.
35. The nucleic acid of paragraph 33, wherein a third vector comprises at least one guide RNA sequence directed towards a gene that encodes a second endogenous protein in the biosynthetic pathway of the least one cannabinoid and at least one sequence encoding an inactive Cas nuclease, and
   wherein, when the first sequence comprises a sequence encoding a transcriptional activator for the gene encoding the first protein, the nucleotide sequence further comprises a transcriptional repressor for the gene encoding the second protein, and
   when the first sequence comprises a sequence encoding a transcriptional repressor for the gene encoding the first protein, the nucleotide sequence further comprises a transcriptional activator for the gene encoding the second protein.
36. The nucleic acid of any one of the paragraphs 24-35, wherein the first protein is CBDAS or THCAS.
37. The nucleic of any one of the paragraphs 24-36, wherein when the first protein is CBDAS, the second protein is THCAS and when the first protein is THCAS, the second protein is CBDAS.

As used herein, including in the claims, singular forms of terms are to be construed as also including the plural form and vice versa, unless the context indicates otherwise. Thus, it should be noted that as used herein, the singular forms "a," "an," and "the" include plural references unless the context clearly dictates otherwise.

Throughout the description and claims, the terms "comprise", "including", "having", and "contain" and their variations should be understood as meaning "including but not limited to" and are not intended to exclude other components.

Those skilled in the art will readily appreciate that all parameters, dimensions, materials, and configurations described herein are meant to be exemplary and that the actual parameters, dimensions, materials, and/or configurations will depend upon the specific application or applications for which the teachings of the present disclosure are used. The present invention also covers the exact terms, features, values and ranges etc. in case these terms, features, values and ranges etc. are used in conjunction with terms such as about, around, generally, substantially, essentially, at least etc. (i.e., "about 3" shall also cover exactly 3 or "substantially constant" shall also cover exactly constant).

The term "at least one" should be understood as meaning "one or more", and therefore includes both embodiments that include one or multiple components. Furthermore, dependent claims that refer to independent claims that describe features with "at least one" have the same meaning, both when the feature is referred to as "the" and "the at least one".

Features disclosed in the specification, unless stated otherwise, can be replaced by alternative features serving the same, equivalent or similar purpose. Thus, unless stated otherwise, each feature disclosed represents one example of a generic series of equivalent or similar features.

Use of exemplary language, such as "for instance", "such as", "for example" and the like, is merely intended to better illustrate the invention and does not indicate a limitation on the scope of the invention unless so claimed. Any steps described in the specification may be performed in any order or simultaneously, unless the context clearly indicates otherwise.

All of the features and/or steps disclosed in the specification can be combined in any combination, except for combinations where at least some of the features and/or steps are mutually exclusive. In particular, preferred features of the invention are applicable to all aspects of the invention and may be used in any combination. The scope of the disclosure is only limited by the appended patent claims.

In the following are provided non-limiting examples of systems for specifically enhancing production of cannabinoids.

### EXAMPLES

### Example 1

A first vector contains a sequence (SEQ ID NO:3) encoding the CBDAS protein sequence (SEQ ID NO:1) regulated by a CaMV 35s promoter (SEQ ID NO:21) and a NOS terminator sequence (SEQ ID NO: 22). In addition, the CBDAS is fused to an 6x Histag (SEQ ID NO:23) to be able to detect the protein for quality control. All these sequences are flanked by a RB T-DNA repeat and LB T-DNA repeat, which are needed for successful introduction of the genetic material into the plant cell (explained hereinafter). This vector also contains the proteins pVs1 StaA (SEQ ID NO: 24), pVs1 RepA (SEQ ID NO:25), that will facilitate BOM sequence (SEQ ID NO: 26) replication. BOM sequence is used for plasmid transfer during bacterial conjugation. The vector contains a pVs1 oriV (SEQ ID NO: 27) to allow vector replication into *Agrobacterium tumefaciens* and another origin of replication (SEQ ID NO:28) for *Escherichia Coli* replication to allow amplification. Finally, inside the vector there is a Kanamycin resistance sequence (SEQ ID NO:29) for conditional selection of the bacteria vector-carriers. This vector has the same backbone as the pCAMBIA0305.2 (SEQ ID NO:30). It is noted however that other vectors carrying the same elements (described in the foregoing description) can alternatively be used as backbones. The final sequence of the vector is provided in SEQ ID NO: 31.

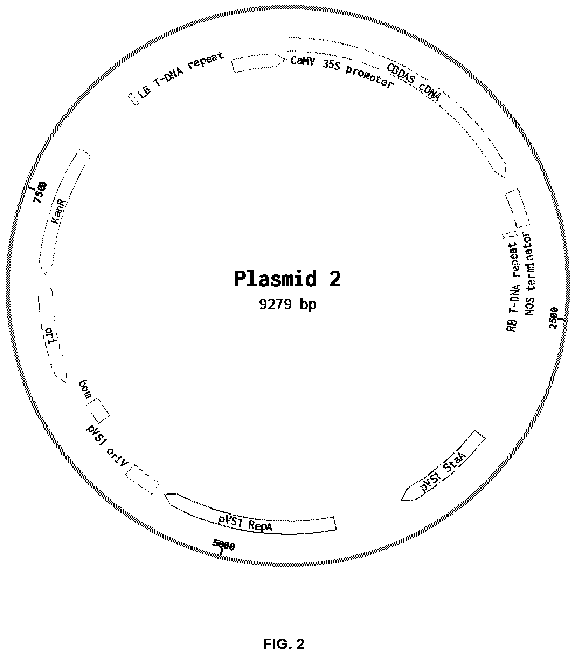

The sequence of the vector was verified by Sanger sequencing, and a vector map ("Plasmid 2") of the vector is shown in FIG. 2.

### Example 2

In a vector (pGreen backbone) constructed for activating the transcriptional machinery, there are different parts. First, flanked by the LB (SEQ ID NO:32) and RB (SEQ ID NO:33) repeats, there is an Atu6-26 promoter (SEQ ID NO:34) which controls the expression of a sgRNA which will guide the machinery into specific regions in the genome. The sgRNAs can be designed towards the endogenous promoter of CBDAS or towards the CaMV35s promoter which will enhance the expression of exogenous CBDAS (i.e. CBDAS that is introduced into the plant) and also the expression of the different dCas9 that is introduced. The sgRNAs designed for this purpose are provided in SEQ ID NO:35., SEQ ID NO:36. and SEQ ID NO:37. In addition, every sgRNA has a scaffold part with a MS2 loop and an f6 aptamer loop (SEQ ID NO:38) to help the recruitment of more TADs into the specific locus. As a terminator for the sgRNA the SUP4 terminator (Seq id no:39) is used. To overexpress the dCas9, a CaMV 35s promoter (SEQ ID NO:40) is additionally used and that controls the expression of a pcoCas9 (SEQ ID NO:41), which has a IV2 intron (SEQ ID NO: 42) that allows the production of pcoCas9 in plants. pcoCas9 is fused to a 2xFLAG tag (SEQ ID NO:43) for quality controls. In the sequence of the pcoCas9 is included a nuclear localization signal (NLS) (SEQ ID NO: 44) to allow the protein to enter the nucleus. The sequence of this protein is followed by a T2A sequence (SEQ ID NO:45) that allows the ribosomal machinery to translate polypeptides. After the T2A sequence, three is the sequence of the MS2 protein (SEQ ID NO:46) fused to the TV technology (6TAL + 2VP64) (SEQ ID NO:47) for transcriptional activation. The terminator for this construct is the NOS terminator (SEQ ID NO:48). Outside the border are the elements needed for bacterial selection, such as the kanamycin resistance gene (SEQ ID NO:49). The sequence of the final vector is provided in SEQ ID NO:50.

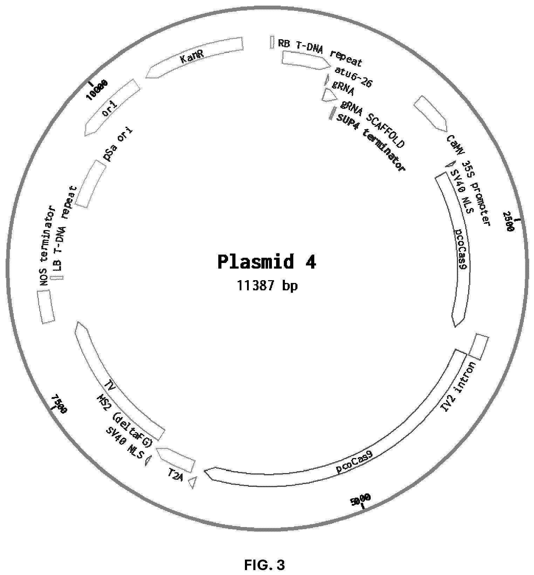

The sequence of the vector was verified by Sanger sequencing, and a vector map ("Plasmid 4") is shown in FIG. 3.

### Example 3

In order to mix the strategy defined with dCas9-Rep, another vector with the same elements as the vector described hereinabove (SEQ ID NO:50) has been built, but with another polypeptide instead of the TAD. This new protein is composed by the KRAB repressor (SEQ ID NO:51), that is a zinc-finger protein that blocks the transcription of the genes. KRAB is fused to a COM protein that recognizes a specific loop of RNA. All the guides in this vector also contain the COM loop in order to guide the dCas9-Rep to the specific loci at the genome. The gRNA sequences are provided in SEQ ID NO:52, SEQ ID NO:53 and SEQ ID NO:54. The vector sequence, confirmed by Sanger sequencing, is provided in SEQ ID NO:55.

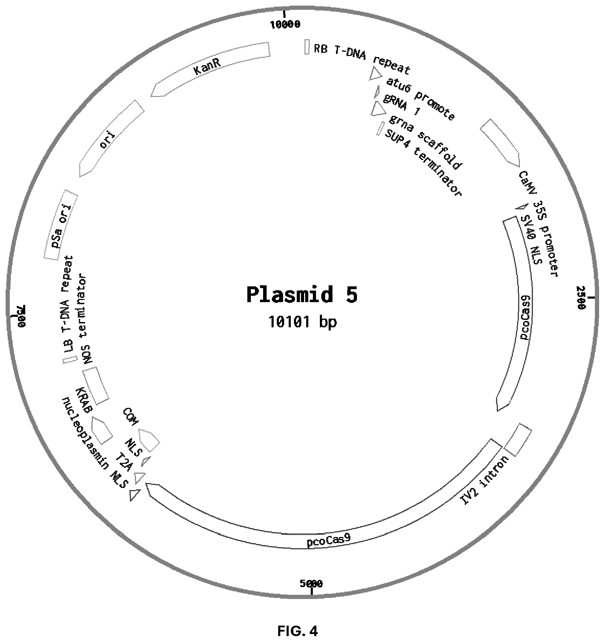

A vector map ("Plasmid 5") is furthermore shown in Fig. 4.

### Example 4

*Efficient agroinfiltration in C sativa analyzed by GUS staining* β-Glucuronidase (GUS) is a very versatile reporter of gene expression that is frequently used in plant molecular biology. The inclusion of the GUS (β-glucuronidase) gene in a plasmid of interest is carried out to study gene expression and the gene's localization in plants. After the plasmid's agroinfiltration in the plants, the histochemical technique of GUS detection can be employed to visualize the gene's activity. This technique involves incubating plant tissue with a substrate that, in the presence of the GUS enzyme, produces a detectable blue reaction product under microscopy. Detecting GUS using this technique provides crucial information about the expression and localization of the gene of interest in plant tissue, which is fundamental for molecular biology studies and crop improvement..

This method is used to ensure the correct agroinfiltration of our plasmid in seedlings (n=4). Seedlings from C sativa were agroinfiltrated using the β-Glucuronidase (GUS) marker, which is a very versatile reporter of gene expression that is frequently used in plant molecular biology. The inclusion of the GUS (β-glucuronidase) gene in a plasmid of interest is carried out to study gene expression and the gene's localization in plants. After the plasmid agroinfiltration in the plants, the histochemical technique of GUS detection can be employed to visualize the gene's activity. This technique involves incubating plant tissue with a substrate that, in the presence of the GUS enzyme, produces a detectable blue reaction product under microscopy.

Using this technique, conditions for an optimal agroinfiltration protocol are set up. Briefly, Agrobacterium Tumefaciens expressing the vector of interest are cultured overnight in LB medium. Next day, the culture at OD600 of 0.6 is used and the cells resuspended in Agroinfiltration media (MS 1x, 10 mM MES, 1% Glucose, 10 mM MgCl2, 400 uM Acetosyringone, pH= 6,8). After 2 hour of incubation, 0.05% pluronic, 0.015% silwett and 5 mM L-ascorbic is added, and then the cells are incubated with the samples (seeds, leaves or flowers) and vacuum applied at 80 mmHg for 10 minutes twice. Samples are then introduced in a plant room with a photoperiod of 16 hour light and 8 hours night and analyzed 3 days after agroinfiltration. All these conditions were carefully tested. In addition, this same protocol was carried out twice with direct injection of the bacterial culture into the plants. This method was also successful and was carried on in the future experiments along with vacuum agroinfiltration. All these agroinfiltration methods showed no visible harm in the plants.

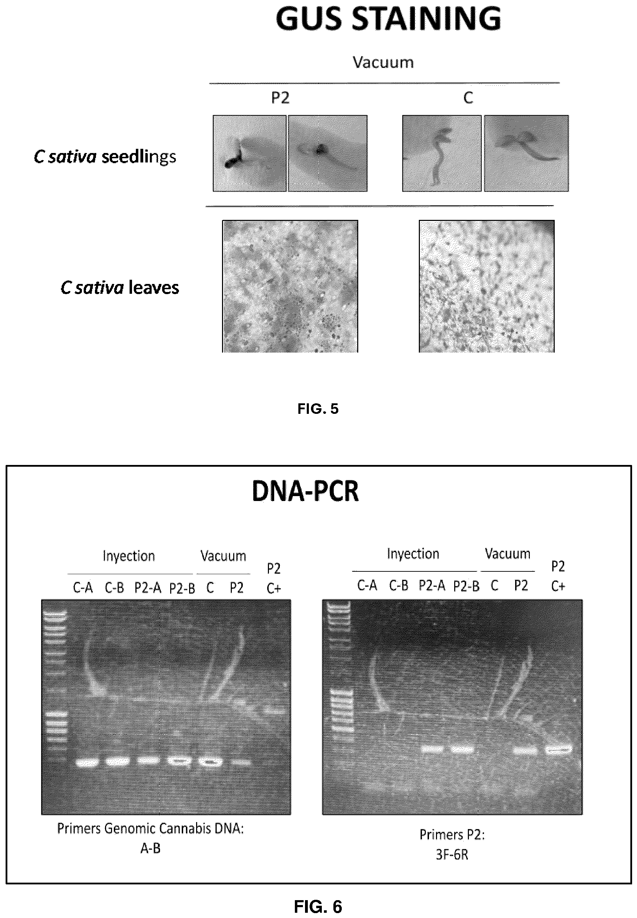

One of the examples of these experiments is shown in Figure 5, where it can be seen that an efficient agroinfiltration in seedlings and leaves agroinfiltrated with a modified version of P2 (plasmid carrying a B-Glucuronidase gene instead CBDAS gene) by an evident bright color in the seedlings and efficient b-gluc staining observed by the microscope in leaves.

Thus, this result shows successful GUS staining in the samples agroinfiltrated with the plasmid of interest (P2; C = control)

### Efficient introduction of vectors in Cannabis sativa seeds, leaves and flowers after agroinfiltration

After setting up the conditions for a successful agroinfiltration in plants, the method was tried with the designed vector. After using the protocol mentioned hereinabove with Plasmid 2 (P2), Plasmid 4 (P4) and Plasmid 5 (P5), the effect of the plasmids was studied. First, the correct introduction of the vectors was confirmed by a PCR strategy. Thus, 3 days after agroinfiltration, genomic DNA was extracted by a Dneasy Mini Plant mini kit and PCR was performed with the primers shown in Table 1 to detect the different genetic constructions that had been introduced.

**Table 1: List of primers used to detect the vectors in plant samples.**

| | |
|---|---|
| Primers P2 | F: TGACGTAAGGGATGACGCAC (SEQ ID NO:68) |
| | R:GTCCTTTCCCGTAGTCCAGC (SEQ ID NO:69) |
| Primers P4 | F: ATGTTGGGGTCAGACGCTTT (SEQ ID NO:70) |
| | R: AGACCGGCAACAGGATTCAA (SEQ ID NO:71) |
| Primers P5 | F: GAGCATCCCACGGAGAAACA (SEQ ID NO:72) |
| | R: AGACCGGCAACAGGATTCAA (SEQ ID NO:73) |

The specific primers (forward and reverse) were also used to detect *Cannabis Sativa.*

Figure 6 shows an example of this PCR strategy to identify a correct agroinfiltration of our plasmids. Specifically the presence of P2 vector was identified (the once including CBDAS cDNA) in the DNA of plant tissue. As a control for the PCR, specific primers that recognize regions only found in Cannabis sativa plants were designed (Left image of the Figure 6). The right image shows the bands resulting from the primers that are going to recognize the specific sequence of plasmid 2, confirming this plasmid has successfully been agroinfiltrated in the sample. The results are consequent with both agroinfiltration methods, and it can be seen that a specific band for P2A and P2B (injection), P2 (vacuum), and P2 C+ are present, thus confirming it corresponds to this unique sequence.

### Efficient expression of our constructs after agroinfiltration at transcriptomics level

After confirmation of an efficient and correct agroinfiltration in plant tissues by GUS staining method and a correct introduction of the plasmids after agroinfiltration, the expression of the effector genes in the transcriptomics of the plant were studied. To this end, several specific primers recognizing the mRNA of the designed constructs were designed.

**Table 2: Primers selected for qPCR analysis.**

| | |
|---|---|
| CBDAS inserted gene | F: CACTTACTTCTCTTCAGTTTTCC (SEQ ID NO:56) |
| | R: ATAGTATCAATCCAGCTCAATT (SEQ ID NO:57) |
| dCAS9 | F:: CTACGTTGGACCACTTGCTAG (SEQ ID NO:58) |
| | R: CTCTCGATGAAAGACTGAGCAG (SEQ ID NO:59) |
| COM-KRAB | F: GTGCGTTATGGCTCCACTAG (SEQ ID NO:60) |
| | R: GTTCTCCAGCATCACATTTCTG (SEQ ID NO:61) |
| MS2-VP64 | F: CCATTCCAATTTTCGCCACG (SEQ ID NO:62) |
| | R: TACCAAATCAGCGTCCATAGG (SEQ ID NO:63) |
| F-box | F: TATCGGCGGAGAGATTTGAG (SEQ ID NO:64) |
| | R: TAAGCCCTTCCCTTGATTCC (SEQ ID NO:65) |

Methods for mRNA extraction and qPCR protocols are explained in the following.

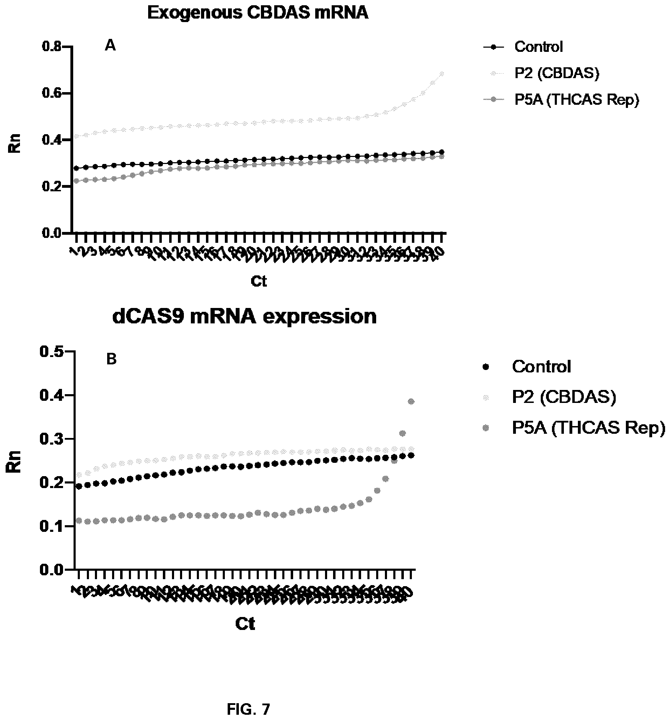

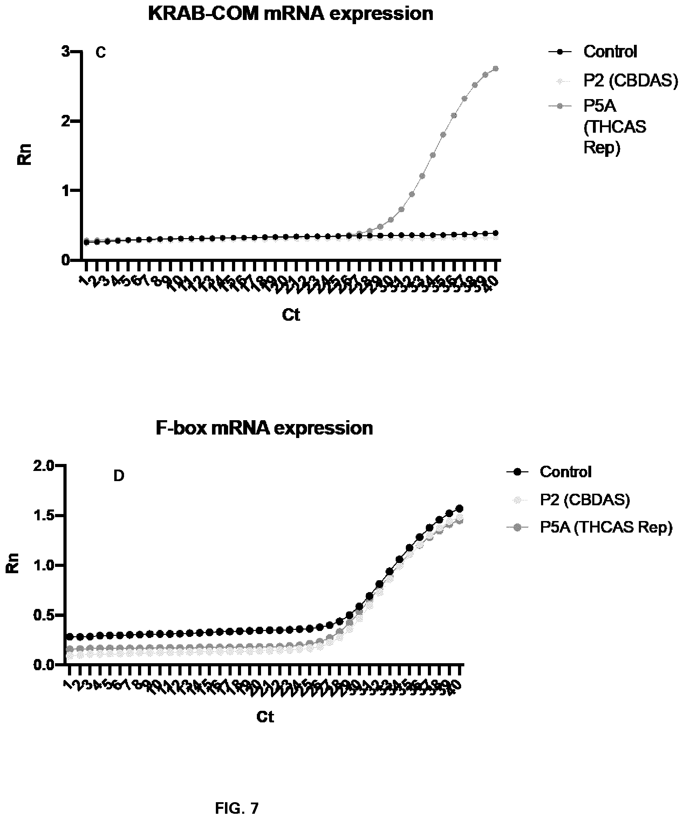

CBDAS exogenous expression was detected only in the samples agroinfiltrated with P2 plasmid (Figure 7A), as it was the only sample with a specific Ct value (35,795). In addition, dCas9 and COM-KRAB fusion mRNA were also detected only in C sativa leaves agroinfiltrated with P5A plasmid (construct designed to promote THCAS transcriptional repression by dCAS9 system) (Figure 7B, 7C). As a housekeeper gene F-box was used, was used, that is a protein structural motif found ubiquitously in cells (Figure 7D). All samples had similar Ct values for the F-box suggesting that RNA extraction and qPCR were correctly performed.

### Efficient overexpression of CBDAS mRNA after method application

The method here described has as its main goal to modulate the quantity of cannabinoids in a *C sativa* plant. One of its main parts is developed by the introduction of a genetic vector that promotes the overexpression of a specific cannabinoid gene. In the experiments, there is focus on overexpressing specifically CBDAS gene. To this aim, a qPCR strategy to detect endogenous mRNA of cannabinoids genes that are modified with the method was designed. Specific primers designed for CBDAS endogenous mRNA are shown in Table 3.

**Table 3: Primers for endogenous CBDAS mRNA.**

| | |
|---|---|
| Total endogenous CBDAS | F: ATGAGAAACTATGGCCTCGC (SEQ ID NO:66) |
| | R: AGTAGACTTTGGGACAGCAAC (SEQ ID NO:67) |

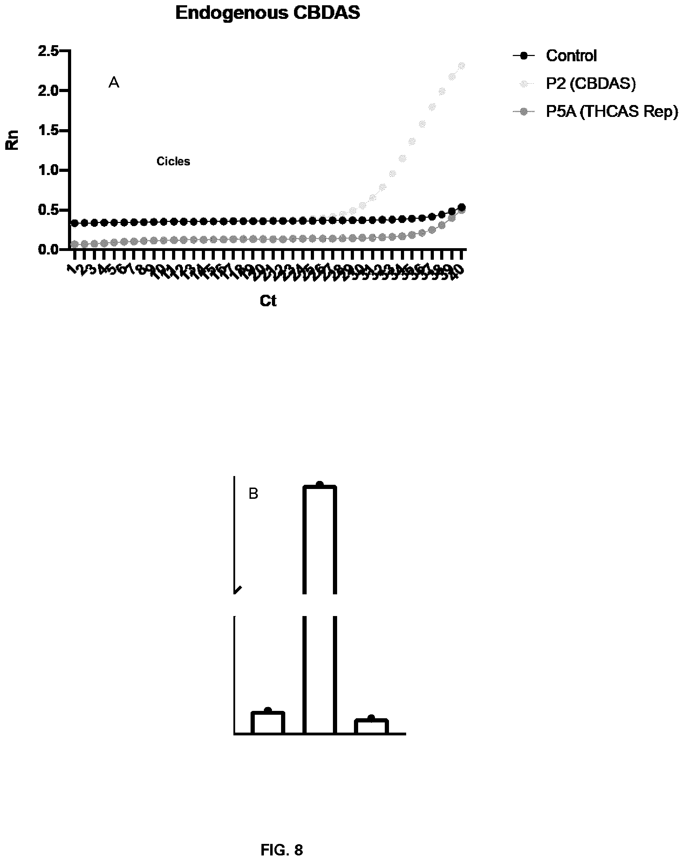

The results indicate that not only is the method capable of expressing this exogenous construct (Figure 7A) but also, a significant upregulation of the CBDAS mRNA total levels is promoted (Figure 8A). By designing specific primers that detect all the mRNAs that contain CBDAS sequence (endogenous + exogenous), it is observed that the plants after P2 plasmid agroinfiltration showed a higher expression of CBDAS transcripts (lower Ct compared with P5 and Control samples) (Figure 8A). Strikingly, it is possible to increase more than 500-fold the levels when compared with control samples and the housekeeping gene as a base (Figure 8B). These results suggest that it is possible to increase CBDAS yield significantly. This number is expected to increase with the addition of the plasmid 4.

In summary, the above results show:
- The methodology allows an efficient delivery in Cannabis sativa plants without affection of its integrity and being totally harmless.
- The application of the methodology in Cannabis sativa plants allows the introduction of desired genetic vectors into the plant tissue.
- The methodology designed is efficiently introduced into the plant cells and processed to promote the expression of the desired transcripts derived from the cDNAs sequences included in the genetic vectors.
- The methodology alters the endogenous transcript levels of the cannabinoid's mRNA of interest.
- The application of the methodology allows an increase of more than 500 times the transcript of the CBDAS gene.

### Example 5

### RNA extraction from plants tissue and qPCR protocol:

Total RNA was obtained using Plant RNeasy Mini Kit (Qiagen), according to the manufacturer's instructions. RNA concentration was measured in a Nanodrop and all samples were diluted to 1µg/µL. cDNA synthesis was performed using Supertranscript II reverse transcriptase (Thermo Fischer Scientific) following manufacturer's protocol. For each sample 1 µg of RNA in 6 µl of H2O was incubated at 65°C for 2 minutes followed by 2 minutes on ice. Afterwards, the following mix was added: 4 µl of 5X buffer, 2 µl of 0.1M DTT, 2 µl of 10 mM dNTPs), 5 µl of random primers (previously diluted 1:30) and 0.5 µl of the SII polymerase (200U/µl). The mixture was incubated at RT for 10 minutes and then 1 hour at 42ºC. The reaction was stopped by heating at 98ºC for 5 minutes. The cDNAs obtained were diluted 20 times and 2 µl were mix with SYBER green PCR Master Mix (Life technologies) and 10 µM) of Fw and Rv primers, in a final volume of 10 µl. The PCR program consisted of an initial step at 95ºC for 10 minutes, followed by 40 cycles of 95ºC for 30 seconds, 56 ºC for 30 seconds and finished by 72 ºC for 1 minute. To detect only expression of the our mRNA, Ct values were analysed. For quantitative analysis, the relative expression of each gene was normalized to F-box as housekeeping gene, according to the formula 2-^{ΔCt}. All samples were analysed with technical triplicates.

## Claims

1. A method of regulating production of at least one cannabinoid in a plant, through gene expression modification of the plant, the method comprising introducing into the plant:
a first nucleotide sequence that is constructed to regulate the endogenous transcriptional machinery of the plant to selectively increase or decrease production of a cannabinoid by the plant, the first nucleotide sequence comprising at least one guide RNA sequence directed towards a plant gene that encodes a first protein in the biosynthetic pathway of the least one cannabinoid, at least one sequence encoding an inactive Cas nuclease and at least one sequence encoding a transcriptional activator or a repressor protein, and
wherein, when the first nucleotide sequence encodes a transcriptional activator the method further comprises introducing a second nucleotide sequence into the plant that is operably linked to a promoter and encodes the first protein;
whereby the level of production of the at least one cannabinoid in the plant is regulated.

2. The method of claim 1, the method further comprising introducing a third nucleotide sequence into the plant, wherein the third nucleotide sequence comprises at least one guide RNA sequence directed towards a gene that encodes a second protein in the biosynthetic pathway of the least one cannabinoid and at least one sequence encoding an inactive Cas nuclease,
wherein, when the first nucleotide sequence comprises a sequence encoding a transcriptional activator for the first protein, the third nucleotide sequence comprises a sequence encoding a transcriptional repressor for the second protein, and wherein when the first nucleotide sequence comprises a sequence encoding a transcriptional repressor for the first protein, the third nucleotide sequence comprises a sequence encoding a transcriptional activator for the second protein.

3. The method any one of the previous claims, wherein the plant is a cannabis plant, such as *Cannabis sativa, Cannabis indica or Cannabis rudelaris.*

4. The method of any one of the previous claims, wherein the first, and/or third nucleotide sequence further comprises at least one guide RNA sequence.

5. The method of the previous claim, wherein the at least one guide RNA sequence encodes an RNA sequence that can be recognized by a recognition protein, such as MS2 coat protein or a COM protein.

6. The method of any one of the previous claims, wherein the sequence encoding a transcriptional activator and/or repressor is fused to the sequence encoding an inactive Cas nuclease and/or a guide RNA binding protein sequence; and/or
wherein the sequence encoding a transcriptional activator, when present, is fused to a sequence encoding a protein that specifically recognizes the at least one guide RNA, such as MS2 coat protein, and
wherein the sequence encoding a the transcriptional repressor, when present, is fused to a sequence encoding a protein that specifically recognizes the at least one guide RNA, such as COM protein.

7. The method of any one of the previous claims, wherein when the first protein is CBDAS, the second protein is THCAS and when the first protein is THCAS, the second protein is CBDAS.

8. The method of any one of the previous claims, wherein the transcriptional activator is selected from AD, AAD, PRD, QAD, STAD, leucine zipper domain, bZIP, zinc finger domain, helix-loop-helix domain, steroid hormone receptor domain, TATA-box binding protein associated factor (TAF) domain, nuclear receptor activation function (AF) domain, VP64, p65, Rta, HSF1, VPR (e.g., VP64, p65, Rta), PH, EDLL, VP16, VP128, TAL,HAT and their combinations and wherein the transcriptional repressor is selected from SRDX-domain containing repressor, KRAB-domain containing repressor, DLN144, DLS, KYP, BRD and MIX.

9. A plant having an increased or decreased content of at least one cannabinoid, the plant comprising a first nucleotide sequence that is constructed to regulate the endogenous transcriptional machinery of the plant to selectively increase or decrease production of the at least one cannabinoid by the plant, the first nucleotide sequence comprising:
at least one guide sequence directed towards a gene that encodes a first protein in the biosynthetic pathway of the least one cannabinoid and at least one sequence encoding an inactive Cas nuclease and at least one sequence encoding a transcriptional activator or a repressor protein, and
wherein, when the first nucleotide sequence is constructed to increase endogenous production of the at least one cannabinoid, the plant further comprise a second nucleotide sequence that is operably linked to a promoter and encodes the first protein in the biosynthetic pathway of the at least one cannabinoid.

10. The plant of the previous claim, wherein the plant further comprises a third nucleotide sequence, wherein the third nucleotide sequence comprises at least one guide RNA sequence directed towards a gene that encodes a second protein in the biosynthetic pathway of the least one cannabinoid and at least one sequence encoding an inactive Cas nuclease, and
wherein, when the first nucleotide sequence comprises a sequence encoding a transcriptional activator for the first protein, the third nucleotide sequence comprises a sequence encoding a transcriptional repressor for the second protein, and wherein the first nucleotide sequence comprises a sequence encoding a transcriptional repressor for the first protein, the third nucleotide sequence comprises a sequence encoding a transcriptional activator for the second protein.

11. The plant of any one of the previous two claims, wherein the plant is a cannabis plant, preferably *cannabis sativa.*

12. A nucleic acid for transforming a plant to produce increased or decreased levels of at least one cannabinoid, the nucleic acid comprising (i) at least one guide RNA sequence directed to a gene encoding a first protein in the biosynthetic pathway of the at least one cannabinoid, (ii) at least one sequence encoding an inactive Cas nuclease and (iii) a first nucleotide sequence encoding a transcriptional activator for the gene and/or at least one nucleotide sequence encoding a transcriptional repressor for the gene, and
wherein, when the first nucleotide sequence encodes a transcriptional activator the nucleic acid further comprises a second nucleotide sequence that is operably linked to a promoter and encodes the first protein.

13. The nucleic acid of the previous claim, further comprising a nucleotide sequence that comprises at least one guide RNA sequence directed towards a gene that encodes a second protein in the biosynthetic pathway of the least one cannabinoid and at least one sequence encoding an inactive Cas nuclease, and
wherein, when the first sequence comprises a sequence encoding a transcriptional activator for the gene encoding the first protein, the nucleic acid further comprises a sequence encoding a transcriptional repressor for the gene encoding the second protein, and when the first sequence comprises a sequence encoding a transcriptional repressor for the gene encoding the first protein, the nucleic acid further comprises a sequence encoding a transcriptional activator for the gene encoding the second protein.

14. The nucleic acid molecule of any one of the previous two claims, wherein the at least one sequence encoding an inactive Cas nuclease is fused to the at least one sequence encoding a transcriptional activator or the transcriptional repressor so as to encode a Cas nuclease fusion protein.

15. The nucleic acid molecule of any one of the previous three claims, wherein the at least one guide RNA sequence contains an RNA sequence that is recognized by a protein that is fused to a transcriptional activator or transcriptional repressor, to thereby enhance or reduce recruitment of the transcriptional machinery.
